# EUROPEAN PATENT SPECIFICATION

(11) **EP 3 378 936 B1**
(45) Date of publication and mention of the grant of the patent: **12.05.2021**
(21) Application number: 17162731.8
(22) Date of filing: 24.03.2017
(51) Int. Cl.: C12N 9/42, C11D 3/386

(54) **CELLULASE SUITABLE FOR USE IN DETERGENT COMPOSITIONS**
CELLULASE ZUR VERWENDUNG IN WASCHMITTELZUSAMMENSETZUNGEN
CELLULASE APPROPRIÉE POUR UNE UTILISATION DANS DES COMPOSITIONS DÉTERGENTES

(43) Date of publication of application: 26.09.2018
(73) Proprietor: Clariant International Ltd, 4132 Muttenz (CH)
(72) Inventor: JAKOB, Claudia, 80992 Muenchen (DE); O'CONNELL, Timothy, 86899 Landsberg am Lech (DE); JOCHENS, Helge, 82065 Baierbrunn (DE); WALLRAPP, Frank, 82131 Gauting (DE); HOESL, Michael, 80469 München (DE); KOHL, Andreas, 82008 Unterhaching (DE); EISELE, Thomas, 82223 Eichenau (DE); BEST, Jonathan, Wirral Merseyside CH63 3JW (GB); COOK, Andrew Thomas, Wirral Merseyside CH63 3JW (GB); KOTSAKIS, Panagiotis, Wirral Merseyside CH63 3JW (GB); LANG, Dietmar Andreas, Wirral Merseyside CH63 3JW (GB); PARRY, Neil James, Wirral Merseyside CH63 3JW (GB); SAMBI, Ilaria, Wirral Merseyside CH63 3JW (GB)
(74) Representative: Graser, Konstanze

(56) References cited:
- WO-A1-2016/066896
- WO-A2-2011/117728
- US-A1- 2005 070 003

### SEQUENCE LISTING

<110> Clariant International Ltd
<120> tbd
<130> tbd
<160> 20
<170> BiSSAP 1.3.6
<210> 1
   <211> 204
   <212> PRT
   <213> Myceliophthora thermophila
<220>
   <223> cellulase
<400> 1
   Gly Ser Gly Gln Thr Thr Arg Tyr Trp Asp Cys Cys Lys Pro Ser Cys
   1               5                   10                  15
   Ala Trp Pro Gly Lys Gly Pro Ser Ser Pro Val Gln Ala Cys Asp Lys
               20                  25                  30
   Asn Asp Asn Pro Leu Asn Asp Gly Gly Ser Thr Arg Ser Gly Cys Asp
           35                  40                  45
   Ala Gly Gly Ser Ala Tyr Met Cys Ser Ser Gln Ser Pro Trp Ala Val
       50                  55                  60
   Ser Asp Glu Leu Ser Tyr Gly Trp Ala Ala Val Lys Leu Ala Gly Ser
   65                  70                  75                  80
   Ser Glu Ser Gln Trp Cys Cys Ala Cys Tyr Glu Leu Thr Phe Thr Ser
                   85                  90                  95
   Gly Pro Val Ala Gly Lys Lys Met Ile Val Gln Ala Thr Asn Thr Gly
               100                 105                 110
   Gly Asp Leu Gly Asp Asn His Phe Asp Leu Ala Ile Pro Gly Gly Gly
           115                 120                 125
   Val Gly Ile Phe Asn Ala Cys Thr Asp Gln Tyr Gly Ala Pro Pro Asn
       130                 135                 140
   Gly Trp Gly Asp Arg Tyr Gly Gly Ile His Ser Lys Glu Glu Cys Glu
   145                 150                 155                 160
   Ser Phe Pro Glu Ala Leu Lys Pro Gly Cys Asn Trp Arg Phe Asp Trp
                   165                 170                 175
   Phe Gln Asn Ala Asp Asn Pro Ser Val Thr Phe Gln Glu Val Ala Cys
               180                 185                 190
   Pro Ser Glu Leu Thr Ser Lys Ser Gly Cys Ser Arg
           195                 200
<210> 2
   <211> 612
   <212> DNA
   <213> Myceliophthora thermophila
<220>
   <223> cellulase
<400> 2
   ggcagcggcc agacgacccg gtactgggac tgctgcaagc cgagctgcgc ctggcccggc      60
   aagggcccct cgtctccggt gcaggcctgc gacaagaacg acaacccgct caacgacggc     120
   ggctccaccc ggtccggctg cgacgcgggc ggcagcgcct acatgtgctc ctcccagagc     180
   ccctgggccg tcagcgacga gctgtcgtac ggctgggcgg ccgtcaagct cgccggcagc     240
   tccgagtcgc agtggtgctg cgcctgctac gagctgacct tcaccagcgg gccggtcgcg     300
   ggcaagaaga tgattgtgca ggcgaccaac accggtggcg acctgggcga caaccacttt     360
   gacctggcca tccccggtgg cggtgtcggt attttcaacg cctgcaccga ccagtacggc     420
   gctcccccga acggctgggg cgaccgctac ggcggcatcc attccaagga agagtgcgaa     480
   tccttcccgg aggccctcaa gcccggctgc aactggcgct tcgactggtt ccaaaacgcc     540
   gacaacccgt cggtcacctt ccaggaggtg gcctgcccgt cggagctcac gtccaagagc     600
   ggctgctccc gt                                                         612
<210> 3
   <211> 137
   <212> PRT
   <213> Streptomyces clavuligerus
<220>
   <223> CBM
<400> 3
   Ala Asp Ala Leu Pro Ala Asp Arg Ala Pro Arg Ala Ala Val Leu Glu
   1               5                   10                  15
   Ala Glu Asp Ala Thr Ile Ser Gln Gly Ala Val Glu Ser Asn His Arg
               20                  25                  30
   Gly Tyr Thr Gly Arg Gly Phe Val Asn Tyr Asp Asn Leu Thr Gly Ser
           35                  40                  45
   Ser Val Glu Trp Thr Val Arg Ala Asp Lys Ala Gly Thr Thr Pro Leu
       50                  55                  60
   Thr Leu Arg Phe Ala Asn Gly Thr Thr Val Asn Arg Pro Met Thr Ile
   65                  70                  75                  80
   Ser Val Asn Gly Thr Pro Ala Leu Thr Gly Arg Ser Phe Pro Gly Asn
                   85                  90                  95
   Gly Ser Trp Thr Ser Trp Gln Thr Ala Asn Leu Asp Val Pro Leu Arg
               100                 105                 110
   Glu Gly Asp Asn Thr Ile Arg Ala Thr Ala Thr Thr Ala Asn Gly Gly
           115                 120                 125
   Pro Asn Val Asp Ser Leu Thr Val Pro
       130                 135
<210> 4
   <211> 411
   <212> DNA
   <213> Streptomyces clavuligerus
<220>
   <223> CBM
<400> 4
<210> 5
   <211> 63
   <212> PRT
   <213> Teredinibacter turnerae
<220>
   <223> linker
<400> 5
   Gly Gly Gly Ser Gly Ser Ser Thr Ser Ser Thr Ser Ser Ser Ser Ser
   1               5                   10                  15
   Ser Ser Ser Ser Ser Ser Ser Ser Ser Ser Ser Ser Ser Ser Ser Ser
               20                  25                  30
   Gly Val Ala Ser Ser Ser Ser Ser Ser Ser Ser Ser Ser Ser Ser Ser
           35                  40                  45
   Ser Ser Ser Ser Ser Ser Ser Ser Ser Ser Ser Ser Gly Ser Gly
       50                  55                  60
<210> 6
   <211> 189
   <212> DNA
   <213> Teredinibacter turnerae
<220>
   <223> linker
<400> 6
   ggcggtggta gcggttcatc gacatcgtct accagttctt caagcagctc gtcttccagc       60
   agttcgtcca gctcttcgag cagttcatcg agctcgggtg tggcgtcatc cagctcgtct      120
   tcgtccagca gcagttcatc gtcgagcagt tcctcctcca gcagcagttc gagctccagt      180
   ggctcaggc                                                              189
<210> 7
   <211> 17
   <212> PRT
   <213> Streptomyces clavuligerus
<220>
   <223> linker
<400> 7
   Asp Ser Gly Gly Pro Gly Pro Gly Pro Gly Pro Gly Pro Gly Gly Thr
   1               5                   10                  15
   Pro
<210> 8
   <211> 51
   <212> DNA
   <213> Streptomyces clavuligerus
<220>
   <223> linker
<400> 8
   gactccgggg gacccggccc cggccccgga cccggcccgg gcggcacccc c 51
<210> 9
   <211> 12
   <212> PRT
   <213> Artificial Sequence
<220>
   <223> catalytic domain motif
<400> 9
   Thr Thr Arg Tyr Trp Asp Cys Cys Lys Pro Ser Cys
   1               5                   10
<210> 10
   <211> 36
   <212> DNA
   <213> Artificial Sequence
<220>
   <223> catalytic domain motif
<400> 10
   acgacccggt actgggactg ctgcaagccg agctgc 36
<210> 11
   <211> 232
   <212> PRT
   <213> Artificial Sequence
<220>
   <223> cellulase
<400> 11
   Val Pro Asp Ser Gly Gly Pro Gly Pro Gly Pro Gly Pro Gly Pro Gly
   1               5                   10                  15
   Gly Thr Pro Gly Ser Gly Gln Thr Thr Arg Tyr Trp Asp Cys Cys Lys
               20                  25                  30
   Pro Ser Cys Ala Trp Pro Gly Lys Gly Pro Ser Ser Pro Val Gln Ala
           35                  40                  45
   Cys Asp Lys Asn Asp Asn Pro Leu Asn Asp Gly Gly Ser Thr Arg Ser
       50                  55                  60
   Gly Cys Asp Ala Gly Gly Ser Ala Tyr Met Cys Ser Ser Gln Ser Pro
   65                  70                  75                  80
   Trp Ala Val Ser Asp Glu Leu Ser Tyr Gly Trp Ala Ala Val Lys Leu
                   85                  90                  95
   Ala Gly Ser Ser Glu Ser Gln Trp Cys Cys Ala Cys Tyr Glu Leu Thr
               100                 105                 110
   Phe Thr Ser Gly Pro Val Ala Gly Lys Lys Met Ile Val Gln Ala Thr
           115                 120                 125
   Asn Thr Gly Gly Asp Leu Gly Asp Asn His Phe Asp Leu Ala Ile Pro
       130                 135                 140
   Gly Gly Gly Val Gly Ile Phe Asn Ala Cys Thr Asp Gln Tyr Gly Ala
   145                 150                 155                 160
   Pro Pro Asn Gly Trp Gly Asp Arg Tyr Gly Gly Ile His Ser Lys Glu
                   165                 170                 175
   Glu Cys Glu Ser Phe Pro Glu Ala Leu Lys Pro Gly Cys Asn Trp Arg
               180                 185                 190
   Phe Asp Trp Phe Gln Asn Ala Asp Asn Pro Ser Val Thr Phe Gln Glu
           195                 200                 205
   Val Ala Cys Pro Ser Glu Leu Thr Ser Lys Ser Gly Cys Ser Arg Gly
       210                 215                 220
   Ser Gly His His His His His His
   225                 230
<210> 12
   <211> 696
   <212> DNA
   <213> Artificial Sequence
<220>
   <223> cellulase
<400> 12
   gttccagact ctggtggtcc aggtccaggt ccaggtccag gtccaggtgg taccccaggt 60
   tctggtcaaa ccactagata ctgggactgt tgtaagccat cttgtgcttg gccaggtaag 120
   ggtccatctt ccccagttca agcttgtgac aagaacgaca acccattgaa cgacggtggt 180
   tctaccagat ccggttgtga cgctggtggt tctgcttaca tgtgttcttc ccaatctcca 240
   tgggctgttt ctgacgagtt gtcttacggt tgggctgccg ttaagttggc tggttcttcc 300
   gagtctcaat ggtgttgtgc ttgttacgag ttgaccttca cttctggtcc agttgctggt 360
   aagaagatga ttgttcaagc taccaacact ggtggtgact tgggtgacaa ccacttcgac 420
   ttggctattc caggtggtgg agttggtatt ttcaacgctt gtaccgacca atacggtgct 480
   ccacctaacg gttggggtga cagatacggt ggtattcact ctaaggagga atgtgagtct 540
   ttcccagagg ctttgaagcc aggttgtaac tggagattcg actggttcca aaacgctgac 600
   aacccatctg ttaccttcca agaggttgct tgtccatctg agttgacctc taagtccggt 660
   tgttctagag gttccggtca tcaccaccat catcac 696
<210> 13
   <211> 404
   <212> PRT
   <213> Artificial Sequence
<220>
   <223> cellulase
<400> 13
   Ala Asp Ala Leu Pro Ala Asp Arg Ala Pro Arg Ala Ala Val Leu Glu
   1               5                   10                  15
   Ala Glu Asp Ala Thr Ile Ser Gln Gly Ala Val Glu Ser Asn His Arg
               20                  25                  30
   Gly Tyr Thr Gly Arg Gly Phe Val Asn Tyr Asp Asn Leu Thr Gly Ser
           35                  40                  45
   Ser Val Glu Trp Thr Val Arg Ala Asp Lys Ala Gly Thr Thr Pro Leu
       50                  55                  60
   Thr Leu Arg Phe Ala Asn Gly Thr Thr Val Asn Arg Pro Met Thr Ile
   65                  70                  75                  80
   Ser Val Asn Gly Thr Pro Ala Leu Thr Gly Arg Ser Phe Pro Gly Asn
                   85                  90                  95
   Gly Ser Trp Thr Ser Trp Gln Thr Ala Asn Leu Asp Val Pro Leu Arg
               100                 105                 110
   Glu Gly Asp Asn Thr Ile Arg Ala Thr Ala Thr Thr Ala Asn Gly Gly
           115                 120                 125
   Pro Asn Val Asp Ser Leu Thr Val Pro Gly Gly Gly Ser Gly Ser Ser
       130                 135                 140
   Thr Ser Ser Thr Ser Ser Ser Ser Ser Ser Ser Ser Ser Ser Ser Ser
   145                 150                 155                 160
   Ser Ser Ser Ser Ser Ser Ser Ser Ser Gly Val Ala Ser Ser Ser Ser
                   165                 170                 175
   Ser Ser Ser Ser Ser Ser Ser Ser Ser Ser Ser Ser Ser Ser Ser Ser
               180                 185                 190
   Ser Ser Ser Ser Ser Gly Ser Gly Gly Ser Gly Gln Thr Thr Arg Tyr
           195                 200                 205
   Trp Asp Cys Cys Lys Pro Ser Cys Ala Trp Pro Gly Lys Gly Pro Ser
       210                 215                 220
   Ser Pro Val Gln Ala Cys Asp Lys Asn Asp Asn Pro Leu Asn Asp Gly
   225                 230                 235                 240
   Gly Ser Thr Arg Ser Gly Cys Asp Ala Gly Gly Ser Ala Tyr Met Cys
                   245                 250                 255
   Ser Ser Gln Ser Pro Trp Ala Val Ser Asp Glu Leu Ser Tyr Gly Trp
               260                 265                 270
   Ala Ala Val Lys Leu Ala Gly Ser Ser Glu Ser Gln Trp Cys Cys Ala
           275                 280                 285
   Cys Tyr Glu Leu Thr Phe Thr Ser Gly Pro Val Ala Gly Lys Lys Met
       290                 295                 300
   Ile Val Gln Ala Thr Asn Thr Gly Gly Asp Leu Gly Asp Asn His Phe
   305                 310                 315                 320
   Asp Leu Ala Ile Pro Gly Gly Gly Val Gly Ile Phe Asn Ala Cys Thr
                   325                 330                 335
   Asp Gln Tyr Gly Ala Pro Pro Asn Gly Trp Gly Asp Arg Tyr Gly Gly
               340                 345                 350
   Ile His Ser Lys Glu Glu Cys Glu Ser Phe Pro Glu Ala Leu Lys Pro
           355                 360                 365
   Gly Cys Asn Trp Arg Phe Asp Trp Phe Gln Asn Ala Asp Asn Pro Ser
       370                 375                 380
   Val Thr Phe Gln Glu Val Ala Cys Pro Ser Glu Leu Thr Ser Lys Ser
   385                 390                 395                 400
   Gly Cys Ser Arg
<210> 14
   <211> 1212
   <212> DNA
   <213> Artificial Sequence
<220>
   <223> cellulase
<400> **14**
   gcggacgccc tccccgcgga ccgcgccccc cgggccgccg tgctcgaggc cgaggacgcg      60
   accatctccc agggcgcggt cgagtccaac caccgcggct acaccggccg gggcttcgtc     120
   aactacgaca acctcaccgg cagcagtgtg gagtggaccg tccgcgccga caaggccggc     180
   accaccccgc tgaccctccg gttcgccaac ggcaccaccg tcaaccgccc gatgaccatc     240
   agcgtcaacg gcaccccggc cctcacgggc cgctccttcc ccggcaacgg gtcctggacg     300
   agctggcaga cggccaacct cgacgtcccc ctcagagagg gcgacaacac gatccgggcc     360
   accgcgacca ccgccaacgg cggccccaac gtcgacagcc tcaccgtccc gggcggtggt     420
   agcggttcat cgacatcgtc taccagttct tcaagcagct cgtcttccag cagttcgtcc     480
   agctcttcga gcagttcatc gagctcgggt gtggcgtcat ccagctcgtc ttcgtccagc     540
   agcagttcat cgtcgagcag ttcctcctcc agcagcagtt cgagctccag tggctcaggc     600
   ggcagcggcc agacgacccg gtactgggac tgctgcaagc cgagctgcgc ctggcccggc     660
   aagggcccct cgtctccggt gcaggcctgc gacaagaacg acaacccgct caacgacggc     720
   ggctccaccc ggtccggctg cgacgcgggc ggcagcgcct acatgtgctc ctcccagagc     780
   ccctgggccg tcagcgacga gctgtcgtac ggctgggcgg ccgtcaagct cgccggcagc     840
   tccgagtcgc agtggtgctg cgcctgctac gagctgacct tcaccagcgg gccggtcgcg     900
   ggcaagaaga tgattgtgca ggcgaccaac accggtggcg acctgggcga caaccacttt     960
   gacctggcca tccccggtgg cggtgtcggt attttcaacg cctgcaccga ccagtacggc    1020
   gctcccccga acggctgggg cgaccgctac ggcggcatcc attccaagga agagtgcgaa    1080
   tccttcccgg aggccctcaa gcccggctgc aactggcgct tcgactggtt ccaaaacgcc    1140
   gacaacccgt cggtcacctt ccaggaggtg gcctgcccgt cggagctcac gtccaagagc    1200
   ggctgctccc gt                                                        1212
<210> 15
   <211> 358
   <212> PRT
   <213> Artificial Sequence
<220>
   <223> cellulase
<400> 15
   Ala Asp Ala Leu Pro Ala Asp Arg Ala Pro Arg Ala Ala Val Leu Glu
   1               5                   10                  15
   Ala Glu Asp Ala Thr Ile Ser Gln Gly Ala Val Glu Ser Asn His Arg
               20                  25                  30
   Gly Tyr Thr Gly Arg Gly Phe Val Asn Tyr Asp Asn Leu Thr Gly Ser
           35                  40                  45
   Ser Val Glu Trp Thr Val Arg Ala Asp Lys Ala Gly Thr Thr Pro Leu
       50                  55                  60
   Thr Leu Arg Phe Ala Asn Gly Thr Thr Val Asn Arg Pro Met Thr Ile
   65                  70                  75                  80
   Ser Val Asn Gly Thr Pro Ala Leu Thr Gly Arg Ser Phe Pro Gly Asn
                   85                  90                  95
   Gly Ser Trp Thr Ser Trp Gln Thr Ala Asn Leu Asp Val Pro Leu Arg
               100                 105                 110
   Glu Gly Asp Asn Thr Ile Arg Ala Thr Ala Thr Thr Ala Asn Gly Gly
           115                 120                 125
   Pro Asn Val Asp Ser Leu Thr Val Pro Asp Ser Gly Gly Pro Gly Pro
       130                 135                 140
   Gly Pro Gly Pro Gly Pro Gly Gly Thr Pro Gly Ser Gly Gln Thr Thr
   145                 150                 155                 160
   Arg Tyr Trp Asp Cys Cys Lys Pro Ser Cys Ala Trp Pro Gly Lys Gly
                   165                 170                 175
   Pro Ser Ser Pro Val Gln Ala Cys Asp Lys Asn Asp Asn Pro Leu Asn
               180                 185                 190
   Asp Gly Gly Ser Thr Arg Ser Gly Cys Asp Ala Gly Gly Ser Ala Tyr
           195                 200                 205
   Met Cys Ser Ser Gln Ser Pro Trp Ala Val Ser Asp Glu Leu Ser Tyr
       210                 215                 220
   Gly Trp Ala Ala Val Lys Leu Ala Gly Ser Ser Glu Ser Gln Trp Cys
   225                 230                 235                 240
   Cys Ala Cys Tyr Glu Leu Thr Phe Thr Ser Gly Pro Val Ala Gly Lys
                   245                 250                 255
   Lys Met Ile Val Gln Ala Thr Asn Thr Gly Gly Asp Leu Gly Asp Asn
               260                 265                 270
   His Phe Asp Leu Ala Ile Pro Gly Gly Gly Val Gly Ile Phe Asn Ala
           275                 280                 285
   Cys Thr Asp Gln Tyr Gly Ala Pro Pro Asn Gly Trp Gly Asp Arg Tyr
       290                 295                 300
   Gly Gly Ile His Ser Lys Glu Glu Cys Glu Ser Phe Pro Glu Ala Leu
   305                 310                 315                 320
   Lys Pro Gly Cys Asn Trp Arg Phe Asp Trp Phe Gln Asn Ala Asp Asn
                   325                 330                 335
   Pro Ser Val Thr Phe Gln Glu Val Ala Cys Pro Ser Glu Leu Thr Ser
               340                 345                 350
   Lys Ser Gly Cys Ser Arg
           355
<210> 16
   <211> 1074
   <212> DNA
   <213> Artificial Sequence
<220>
   <223> cellulase
<400> 16
   gcggacgccc tccccgcgga ccgcgccccc cgggccgccg tgctcgaggc cgaggacgcg      60
   accatctccc agggcgcggt cgagtccaac caccgcggct acaccggccg gggcttcgtc     120
   aactacgaca acctcaccgg cagcagtgtg gagtggaccg tccgcgccga caaggccggc     180
   accaccccgc tgaccctccg gttcgccaac ggcaccaccg tcaaccgccc gatgaccatc     240
   agcgtcaacg gcaccccggc cctcacgggc cgctccttcc ccggcaacgg gtcctggacg     300
   agctggcaga cggccaacct cgacgtcccc ctcagagagg gcgacaacac gatccgggcc     360
   accgcgacca ccgccaacgg cggccccaac gtcgacagcc tcaccgtccc ggactccggg     420
   ggacccggcc ccggccccgg acccggcccg ggcggcaccc ccggcagcgg ccagacgacc     480
   cggtactggg actgctgcaa gccgagctgc gcctggcccg gcaagggccc ctcgtctccg     540
   gtgcaggcct gcgacaagaa cgacaacccg ctcaacgacg gcggctccac ccggtccggc     600
   tgcgacgcgg gcggcagcgc ctacatgtgc tcctcccaga gcccctgggc cgtcagcgac     660
   gagctgtcgt acggctgggc ggccgtcaag ctcgccggca gctccgagtc gcagtggtgc     720
   tgcgcctgct acgagctgac cttcaccagc gggccggtcg cgggcaagaa gatgattgtg     780
   caggcgacca acaccggtgg cgacctgggc gacaaccact ttgacctggc catccccggt     840
   ggcggtgtcg gtattttcaa cgcctgcacc gaccagtacg gcgctccccc gaacggctgg     900
   ggcgaccgct acggcggcat ccattccaag gaagagtgcg aatccttccc ggaggccctc     960
   aagcccggct gcaactggcg cttcgactgg ttccaaaacg ccgacaaccc gtcggtcacc    1020
   ttccaggagg tggcctgccc gtcggagctc acgtccaaga gcggctgctc ccgt          1074
<210> 17
   <211> 365
   <212> PRT
   <213> Artificial Sequence
<220>
   <223> cellulase
<400> 17
   Ala Asp Ala Leu Pro Ala Asp Arg Ala Pro Arg Ala Ala Val Leu Glu
   1               5                   10                  15
   Ala Glu Asp Ala Thr Ile Ser Gln Gly Ala Val Glu Ser Asn His Arg
               20                  25                  30
   Gly Tyr Thr Gly Arg Gly Phe Val Asn Tyr Asp Asn Leu Thr Gly Ser
           35                  40                  45
   Ser Val Glu Trp Thr Val Arg Ala Asp Lys Ala Gly Thr Thr Pro Leu
       50                  55                  60
   Thr Leu Arg Phe Ala Asn Gly Thr Thr Val Asn Arg Pro Met Thr Ile
   65                  70                  75                  80
   Ser Val Asn Gly Thr Pro Ala Leu Thr Gly Arg Ser Phe Pro Gly Asn
                   85                  90                  95
   Gly Ser Trp Thr Ser Trp Gln Thr Ala Asn Leu Asp Val Pro Leu Arg
               100                 105                 110
   Glu Gly Asp Asn Thr Ile Arg Ala Thr Ala Thr Thr Ala Asn Gly Gly
           115                 120                 125
   Pro Asn Val Asp Ser Leu Thr Val Pro Asn Asp Asp Pro Leu Pro Gly
       130                 135                 140
   Lys Asn Thr Pro Thr Pro Thr Pro Thr Thr Ser Ala Pro Leu Ser Ser
   145                 150                 155                 160
   Gly Gly Ser Gly Gln Thr Thr Arg Tyr Trp Asp Cys Cys Lys Pro Ser
                   165                 170                 175
   Cys Ala Trp Pro Gly Lys Gly Pro Ser Ser Pro Val Gln Ala Cys Asp
               180                 185                 190
   Lys Asn Asp Asn Pro Leu Asn Asp Gly Gly Ser Thr Arg Ser Gly Cys
           195                 200                 205
   Asp Ala Gly Gly Ser Ala Tyr Met Cys Ser Ser Gln Ser Pro Trp Ala
       210                 215                 220
   Val Ser Asp Glu Leu Ser Tyr Gly Trp Ala Ala Val Lys Leu Ala Gly
   225                 230                 235                 240
   Ser Ser Glu Ser Gln Trp Cys Cys Ala Cys Tyr Glu Leu Thr Phe Thr
                   245                 250                 255
   Ser Gly Pro Val Ala Gly Lys Lys Met Ile Val Gln Ala Thr Asn Thr
               260                 265                 270
   Gly Gly Asp Leu Gly Asp Asn His Phe Asp Leu Ala Ile Pro Gly Gly
           275                 280                 285
   Gly Val Gly Ile Phe Asn Ala Cys Thr Asp Gln Tyr Gly Ala Pro Pro
       290                 295                 300
   Asn Gly Trp Gly Asp Arg Tyr Gly Gly Ile His Ser Lys Glu Glu Cys
   305                 310                 315                 320
   Glu Ser Phe Pro Glu Ala Leu Lys Pro Gly Cys Asn Trp Arg Phe Asp
                   325                 330                 335
   Trp Phe Gln Asn Ala Asp Asn Pro Ser Val Thr Phe Gln Glu Val Ala
               340                 345                 350
   Cys Pro Ser Glu Leu Thr Ser Lys Ser Gly Cys Ser Arg
           355                 360                 365
<210> 18
   <211> 1095
   <212> DNA
   <213> Artificial Sequence
<220>
   <223> cellulase
<400> 18 agcggctgct cccgt 1095
<210> 19
   <211> 15
   <212> PRT
   <213> Artificial Sequence
<220>
   <223> carbohydrate binding domain tag
<400> 19
   Val Pro Asp Ser Gly Gly Pro Gly Pro Gly Pro Gly Pro Gly Pro
   1               5                   10                  15
<210> 20
   <211> 45
   <212> DNA
   <213> Artificial Sequence
<220>
   <223> carbohydrate binding domain tag
<400> 20
   gttccagact ctggtggtcc aggtccaggt ccaggtccag gtcca 45

## Description

The present invention relates to a novel cellulase, the use of the novel cellulase in various applications such as detergent compositions and a composition comprising the novel cellulase.

Cellulases are used for various applications and are of particular importance for the good performance of cleaning applications such as the performance of detergent compositions.

There are various cellulases known within the art, however, functional requirements are constantly increasing as customers expect high cleaning performance while production costs and thus costs of the final product should be kept low. In addition, environmental aspects gain importance and requirements for marketing approval constantly increase. So far, whiteness of fabrics is often achieved by implementation of bleaching agents such as chlorine-containing compositions. This leads not only to damages of the fabrics but is also increasing costs and environmentally harmful. Such state of the art enzymes are for example disclosed within US 2005 070003 A1.

US20050070003 discloses recombinant cellulases which comprise a catalytic domain of *M. thermophila* and a cellulase binding domain from *H.insolens* which are discussed for washing applications.

WO2011/117728 discloses fusion proteins comprising cellulases with various catalytic linker and carbohydrate binding domains which are used within the production of ethanol from lignocellulosic biomass.

WO2016/066896 discloses genetically modified endoglucanases and their effects in textile treatment and detergent applications.

The inventors of the present invention have therefore set themselves the task to develop a novel cellulase suitable for use within detergent compositions, which shows significantly improved properties in view of the cellulases known in the art. It has been of particular importance to the inventors of the present invention that whiteness of fabrics was increased due to an improved ARD (Anti Re-Deposition; The term "Anti Re-Deposition" and the respective method is defined within the examples) effect while damage or degeneration of the fabric is minimalized or can even be avoided.

The inventors of the present invention have now surprisingly found that this task can be met by a cellulase comprising the catalytic domain motif [STA]-T-R-Y-[FYW]-D-x(5)-[CA] and a carbohydrate binding domain with a sequence identity of at least 80% to SEQ ID NO: 3 or a carbohydrate binding domain comprising the tag motif V-[PSC]-[DQEN]-S-G-G-P-G-P-G-P-G-P-G-P and wherein the cellulase has a sequence identity of at least 99% to SEQ ID NO: 11, SEQ ID NO: 13, SEQ ID NO: 15 or SEQ ID NO: 17.

The cellulase of the present invention does not only show excellent cleaning performance and a significantly increased whiteness of fabrics but also an outstanding de-pilling effect. To date detergent and particularly laundry compositions frequently contain cellulase blends (i.e. a cellulase for whiteness and a further cellulase for depilling). This increases the enzyme content of the detergent, the cost per dose and the amount of enzyme rinsed away at the end of a wash cycle. Furthermore, the different cellulases may have different stability and activity requirements and optima, making formulation and wash instructions more difficult.

In addition, the inventive cellulase shows excellent whiteness (ARD) and de-pilling effects even at low temperatures such as temperatures of 40 °C , 35 °C or even below 35 °C, preferably below 25°C and up to 20 °C and below 20°C.

A further advantage of the inventive cellulase is a high expression rate of the inventive cellulase further decreasing production costs.

Within the present invention, the term "cellulase" is to be understood as referring to any enzyme catalyzing cellulolysis, which is the decomposition of cellulose and related polysaccharides. Within the present invention, the term "cellulase" also refers to any naturally occurring mixture or complex of such enzymes, which act serially or synergistically to decompose cellulosic material. The cellulase of the present invention may be of fungal, bacterial or protozoal origin. The term "cellulases" refers in particular to any enzyme capable of breaking down cellulose into monosaccharides such as beta-glucose, or shorter polysaccharides and oligosaccharides.

The term "de-pilling" refers to the ability of cellulase enzymes to remove cotton fuzz and loose surface fibers in or on the fabric. This process is also referred to as "depilling", "biopolishing" and "biofinishing" and smoothes the surface of the fabric, which in turn improves its softness and appearance. Cellulase treatment also aids in the prevention of subsequent formation of fiber pills that make the garments appear worn. During de-pilling it is desirable to minimize strength loss of the fabric due to the hydrolytic action of the cellulases.

The inventive cellulase comprises the catalytic domain motif [STA]-T-R-Y-[FYW]-D-x(5)-[CA]. Any motif or modification as referred to within the present application is defined using the one letter code for amino acids well known to a person skilled in the art.

The amino acids are encoded as follows:
G Glycine, P Proline, A Alanine, V Valine, L Leucine, I Isoleucine, M Methionine, C Cysteine, F Phenylalanine, Y Tyrosine, W Tryptophan, H Histidine, K Lysine, R Arginine, Q Glutamine, N Asparagine, E Glutamic Acid, D Aspartic Acid, S Serine, T Threonine.

The amino acids in square brackets are to be understood as alternatives of the respective position. The "x" indicates that the the respective position may be selected from all existing amino acids. The number in parenthesis (within this definition referred to as variable "z") (e.g. 5 as contained within the catalytic domain motif) indicates that the term ("term" meaning amino acid or any motif such as [ACT]) in front of the parenthesis is repeated z times (e.g. A(5) indicates that the amino acid A is repeated 5 times). In case there are two numbers indicated e.g. (5, 10), the term (as defined above) in front of the parenthesis may be repeated from 5 to 10 times.

Within a preferred embodiment of the present invention, the catalytic domain motif is T-T-R-Y-[FYW]-D-x(5)-[CA].

Within a particularly preferred embodiment of the present invention, the catalytic domain motif is T-T-R-Y-W-D-x(5)-C wherein the catalytic domain motif T-T-R-Y-W-D-C-C-K-P-S-C (also referred to as SEQ ID NO: 9) is most preferred.

The cellulase of the present invention further comprises a carbohydrate binding domain with a sequence identity of at least 80%, preferably at least 85%, further preferred at least 90%, even more preferred at least 92%, also preferred at least 95%, particularly preferred at least 98% and most preferred at least 99% to SEQ ID NO: 3 or a carbohydrate binding tag motif V-[PSC]-[DQEN]-S-G-G-P-G-P-G-P-G-P-G-P, wherein a carbohydrate binding domain tag of V-P-D-S-G-G-P-G-P-G-P-G-P-G-P is most preferred.

Within the present invention, the term "carbohydrate binding domain tag" refers to any sequence comprising the motif V-[PSC]-[DQEN]-S-G-G-P-G-P-G-P-G-P-G-P which might be directly connected to the cellulase catalytic domain or via a linker, preferably a linker as defined herein.

The sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues X 100)/(Length of Alignment - Total Number of Gaps in Alignment)

Within a particularly preferred embodiment, the inventive cellulase comprises comprising a sequence with at least 80%, preferably at least 85%, further preferred at least 90%, even more preferred at least 92%, also preferred at least 95%, particularly preferred at least 98% and most preferred at least 99% sequence identity to SEQ ID NO: 1.

Within a preferred embodiment, the inventive cellulase further comprises a linker. The term "linker" refers to any sequence known to a person skilled in the art as suitable to connect or "link" the catalytic domain and the carbohydrate binding domain or the carbohydrate binding domain tag. "Linkers" or "spacers" are short amino acid sequences created in nature to separate multiple domains in a single protein. The function to prohibit unwanted interactions between the catalytic domain and the carbohydrate binding domain or the carbohydrate binding domain tag without interfering with the function of each domain. Some linkers have been surprisingly found to contribute to the performance of the inventive cellulase and to increase ARD (Anti Re-Deposition) and de-pilling effects and the inventors of the present invention have even been able to identify a linker motif.

Within a preferred embodiment, the linker has a sequence identity of at least 80%, preferably at least 85%, further preferred at least 90%, even more preferred at least 92%, also preferred at least 95%, particularly preferred at least 98% and most preferred at least 99% to SEQ NO: 5. It is thereby further preferred that the linker comprises an amino acid sequence of [AGSVT](5,65).

Within another preferred embodiment, the linker has a sequence identity of at least 80%, preferably at least 85%, further preferred at least 90%, even more preferred at least 92%, also preferred at least 95%, particularly preferred at least 98% and most preferred at least 99% to SEQ NO: 7. It is thereby further preferred that the linker comprises an amino acid sequence of ([SG]-P) (5,10).

The inventive cellulase is selected from any sequence with a sequence identity of at least 99% to SEQ ID NO: 11, SEQ ID NO: 13, SEQ ID NO: 15 or SEQ ID NO: 17.

The inventive cellulase may be prepared by any method known to a person skilled in the art as suitable for the inventive purpose. Preferred is the expression of the inventive cellulase by a suitable host cell. The term "host cell" refers to any cell type that is suitable to transformation, transfection, transduction, or the like with a nucleic acid construct or expression vector comprising the nucleic acid sequence of the inventive cellulase. The term "host cell" also encompasses any progeny of a parent cell, which is not identical to the parent cell due to mutations that occur during replication. Host cells are preferably selected from the group consisting of fungi, yeast and bacteria. Within the present invention, the host cell is preferably a yeast cell such as *Candida, Hansenula, Kluyveromyces; Pichia, Saccharomyces, Schizosaccharomyces,* or *Yarrowia* wherein *Kluyveromyces lactis, Kaccharomyces carlsbergensis, Saccharomyces cerevisiae, Saccharomyces diastaticus, Saccharomyces douglasii, Saccharomyces kluyveri, Saccharomyces norbensis, Saccharomyces oviformis, Yarrowia Iipolytica* and *Pichia pastoris* are preferred.

The term "expression" includes any step involved in the production of the inventive cellulase such as but not limited to transcription, posttranscriptional, modification, translation, post-translational modification and secretion.

Within another aspect, the present invention relates to the use of the inventive cellulase as defined within the application in textile, feed, food, biomass hydrolysis, plant oil refining, detergent and pulp and paper processing applications. Within a preferred embodiment, the inventive cellulase is used as a biofinishing agent such as a depilling agent or a defuzzing agent; a biostoning agent; a fabric care agent such as a color clarification agent, an anti greying agent, a softness agent, an antipilling agent; a laundry agent such as an an anti-redeposition agent, a soil removal agent; a malting and brewing agent such as a filtration agent, a color extraction agent; a bakery agent such as a texture agent; a feed agent such as a viscosity reduction agent; a fruit processing agent such as a release antioxidants agent, a pressing agent, a color extraction agent, a clarification of juice agent; a plant oil extraction agent; a pulp and paper processing agent such as a deinking agent, a fiber brightness agent, a drainage agent or a bleaching agent.

Textile applications such as the use in cleaning and laundry applications are preferred. It is thereby particularly preferred to use the inventive cellulase as a component of a composition for use in laundry and cleaning applications such as a detergent composition.

Within another aspect the present invention relates to a composition comprising at least one inventive cellulase. Within a preferred embodiment, the composition comprises at least one surfactant, builder, co-builder, anti-redeposition agent, dispersant, optical brightener, bleaching agent, dye, enzyme, chelator, polymer, pH buffering agent, filler, flocculant, fabric softener, foam booster, perfume, suds supressor, bacteriocide, fungicide, enzyme inhibitor, stabilizer, solubilizer, antioxidant, anti-corrosion agent, shading dye and/or pigment. Within a particularly preferred embodiment, the inventive composition is a detergent composition for textile(s) or textile material or a non-fabric detergent composition.

The detergent composition of the invention may be in any convenient form, such as a bar, a homogenous tablet, a tablet having two or more layers, a pouch having one or more compartments, a regular or compact powder, a granule, a paste, a gel, or a regular, compact or concentrated liquid.

Pouches can be configured as single or multicompartments. It can be of any form, shape and material which is known to a person skilled in the art as suitable for the inventive purpose and which prevents the release of the composition from the pouch prior to water contact. The pouch is preferably made from water soluble film which encloses an inner volume. The inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, polymethacrylates, most preferably polyvinyl alcohol copolymers and, hydroxyprpyl methyl cellulose (HPMC).

A liquid or gel detergent may be aqueous, preferably containing at least 20% by weight and up to 95 weight-% water, such as up to about 70 weight-% water, up to about 65 weight-% water, up to about 55 weight-% water, up to about 45 weight-% water, up to about 35 weight-% water, wherein a water content of from 2 to 40 weight-% is preferred. Other types of liquids include alkanols, amines, diols, ethers and polyols in an aqueous liquid or gel. An aqueous liquid or gel detergent may contain from 0 to 30 weight-% of an organic solvent. A liquid or gel detergent may also be non-aqueous.

The term "textile" refers to woven fabrics, as well as staple fibers and filaments suitable for conversion to or use as yarns, woven, knit, and non-woven fabrics. The term encompasses yarns made from natural, as well as synthetic (e.g., manufactured) fibers. The term, "textile materials" is a general term for fibers, yarn intermediates, yarn, fabrics, and products made from fabrics (e.g., garments and other articles).

The term "non-fabric detergent compositions" include non-textile surface detergent compositions, including but not limited to compositions for hard surface cleaning, such as dishwashing detergent compositions, oral detergent compositions, denture detergent compositions, and personal cleansing compositions
A composition for use in laundry liquid may include from 0.0001 to 10 weight-%, preferably from 0.0005 to 8 weight.-% such as from 0.001 to 5 weight-%, preferably of from 0.002 to 2 weight-%, particularly preferred of from 0.003 to 1 weight-% of the inventive cellulase relative to the weight of the composition wherein ranges of from 0.0005 to 0.5 weight-%, for example from 0.0005 to 0.05 weight-% are also preferred.

The inventive cellulase may be stabilized using conventional stabilizing agents for example a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, peptide aldehydes or ketones.

Within a preferred embodiment, the composition comprises a shading dye. Preferably, the shading dye is a blue or violet shading dye, although other hues are also within the scope of the invention. The use of a blue or violet shading dye may enhance the perception of whiteness and / or cleanness.

Suitable shading dyes are described in WO 2011/047987, the contents of which are incorporated herein by reference in their entirety.

Preferably, the shading dye is a reactive dye covalently bound to a polymer. More preferably, the polymer is a polyimine, optionally but preferably wherein the polyimine is substituted with 2-hydroxypropan-1-yl groups.

The shading dye may preferably comprise a chromophore of formula: 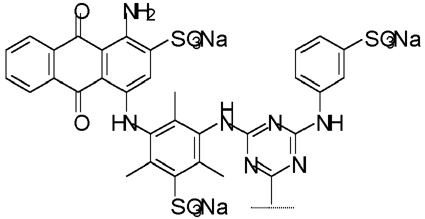 wherein ---- represents a point of attachment.

An exemplary and preferred shading dye is UB40, which has the following structure:

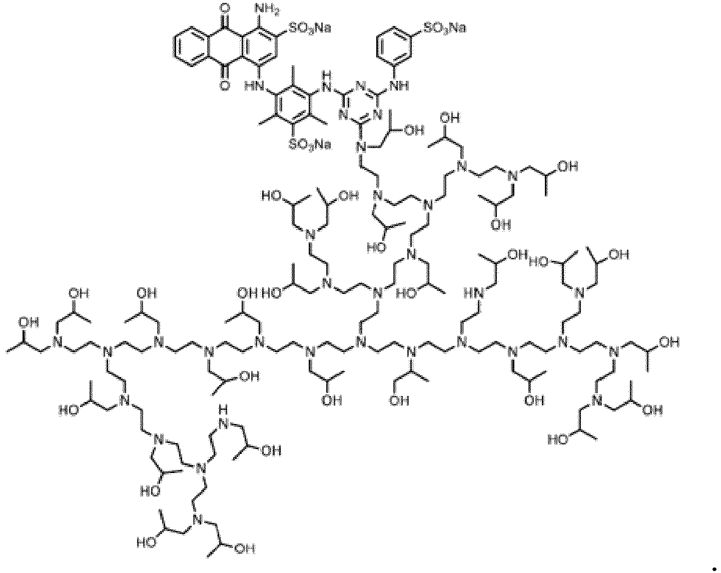

In some cases, the shading dye is preferably a direct dye, for example, an azine dye.

Suitable azine dyes are described in WO 2008/017570, the contents of which are incorporated herein by reference in their entirety.

In some cases, the dye is preferably AV50, which has the following structure:

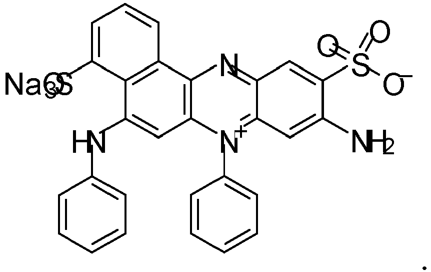

The shading dye is preferably present in the composition in an amount of from 0.0001 to 1 weight-%, more preferably from 0.0001 to 0.1 weight- %, even more preferably from 0.0001 to 0.01 weight- %, most preferably from 0.0005 to 0.001 weight-%.

Within a preferred embodiment, the composition comprises from 0.1 to 80 weight-%, preferably from 1 to 70 weight-%, more preferably from 2 to 60 weight-%, particularly preferred of from 4 to 40 weigh-%, even more preferred of from 5 to 35 weight-% and most preferred of from 6 to 33 weight-% of a surfactant.

Suitable surfactants may be chosen from the surfactants described "Surface Active Agents" Vol. 1, by Schwartz & Perry, Interscience 1949, Vol. 2 by Schwartz, Perry & Berch, Interscience 1958, in the current edition of "McCutcheon's Emulsifiers and Detergents" published by Manufacturing Confectioners Company or in "Tenside-Taschenbuch", H. Stache, 2nd Edn., Carl Hauser Verlag, 1981 or in Anionic Surfactants: Organic Chemistry edited by Helmut W. Stache (Marcel Dekker 1996) .

Within a preferred embodiment, the inventive composition comprises at least one anionic detergent compound (or anionic surfactant) preferably selected from water-soluble alkali metal salts of organic sulphates and sulphonates having alkyl radicals containing from about 8 to about 22 carbon atoms, the term alkyl being used to include the alkyl portion of higher alkyl radicals.

Examples of suitable synthetic anionic detergent compounds are sodium and potassium alkyl sulphates, especially those obtained by sulphating higher C₈ to C₁₈ alcohols, produced for example from tallow or coconut oil, alkyl ether carboxylic acids; sodium and potassium alkyl C₉ to C₂₀ benzene sulphonates, particularly sodium linear secondary alkyl C₁₀ to C₁₅ benzene sulphonates; and sodium alkyl glyceryl ether sulphates, especially those ethers of the higher alcohols derived from tallow or coconut oil and synthetic alcohols derived from petroleumand is preferably selected from linear alkyl benzene sulphonate; alkyl sulphates; alkyl ether sulphates; alkyl ether carboxylates; soaps; alkyl (preferably methyl) ester sulphonates, and mixtures thereof. Particularly preferred are alkyl ether sulphates such as C₁₂-C₁₄ n-alkyl ether sulphates with an average of 1 to 3EO (ethoxylate) units. Sodium lauryl ether sulphate is particularly preferred (SLES). Preferably the linear alkyl benzene sulphonate is a sodium C₁₁ to C₁₅ alkyl benzene sulphonates. Preferably the alkyl sulphates is a linear or branched sodium C₁₂ to C₁₈ alkyl sulphates. Sodium dodecyl sulphate is particularly preferred (SDS, also known as primary alkyl sulphate).

Within a preferred embodiment two or more anionic detergent compounds are present within the composition, for example linear alkyl benzene sulphonate together with an alkyl ether sulphate.

Within a particularly preferred embodiment, the anionic detergent compound is selected from linear alkyl benzene sulphonates; alkyl sulphates; alkyl ether sulphates; and mixtures thereof.

The composition may comprise anionic and/or non-ionic detergent compounds (surfactants).

Within a preferred embodiment, the composition contains at least one nonionic detergent compound (or surfactant) preferably selected from the reaction products of compounds having an aliphatic hydrophobic group and a reactive hydrogen atom, for example, aliphatic alcohols, acids or amides, especially ethylene oxide either alone or with propylene oxide. Specific nonionic detergent compounds are the condensation products of aliphatic C₈ to C₁₈ primary or secondary linear or branched alcohols with ethylene oxide. Preferably the alkyl ethoxylated non-ionic surfactant is a C₈ to C₁₈ primary alcohol with an average ethoxylation of 7EO to 9EO units.

If a non-ionic surfactant is present, then most preferably the non-ionic surfactant is an alcohol ethoxylate, more preferably a C₁₀-C₁₈ alcohol ethoxylate having an average of 3-10 moles of ethylene oxide, most preferably a C₁₂-C₁₅ alcohol ethoxylate having an average of 5-9 moles of ethylene oxide.

Within a particularly preferred embodiment, the inventive composition comprises a surfactant composition comprising of from 4 to 40 weight-%, more preferably from 5 to 35 weight-%, most preferably from 6 to 33 weight-% of a surfactant that comprises an anionic surfactant, preferably comprising linear alkyl benzene sulphonates and nonionic surfactant. In this preferable surfactant mixture, most preferably the weight fraction of nonionic surfactant to anionic surfactant is < 0.5, for example from 0.1 to < 0.5. This means that it is preferable that the level of anionic surfactant is greater than the level of nonionic surfactant in the detergent composition.

Within a preferred embodiment, the inventive composition preferably comprises a perfume. Preferably the perfume comprises at least one note (compound) from: alpha-isomethyl ionone, benzyl salicylate; citronellol; coumarin; hexyl cinnamal; linalool; Pentanoic acid, 2-methyl-, ethyl ester; octanal; benzyl acetate; 1,6-octadien-3-ol, 3,7-dimethyl-, 3-acetate; cyclohexanol, 2-(1,1-dimethylethyl)-, 1-acetate; delta-damascone; beta-ionone; verdyl acetate; dodecanal; hexyl cinnamic aldehyde; cyclopentadecanolide; benzeneacetic acid, 2-phenylethyl ester;amyl salicylate; beta-caryophyllene; ethyl undecylenate; geranyl anthranilate; alpha-irone; beta-phenyl ethyl benzoate; alpa-santalol; cedrol; cedryl acetate; cedry formate; cyclohexyl salicyate; gamma-dodecalactone; and, beta phenylethyl phenyl acetate.

Within a preferred embodiment, the inventive composition preferably comprises a fluorescent agent (optical brightener). Fluorescent agents are well known and many such fluorescent agents are available commercially. Usually, these fluorescent agents are supplied and used in the form of their alkali metal salts, for example, the sodium salts.

Preferred classes of fluorescers are: Di-styryl biphenyl compounds, e.g. Tinopal (Trade Mark) CBS-X, Di-amine stilbene di-sulphonic acid compounds, e.g. Tinopal DMS pure Xtra and Blankophor (Trade Mark) HRH, and Pyrazoline compounds, e.g. Blankophor SN.

Preferred fluorescers are: sodium 2 (4-styryl-3-sulphophenyl)-2H-napthol[1,2-d]triazole, disodium 4,4'-bis{[(4-anilino-6-(N methyl-N-2 hydroxyethyl) amino 1,3,5-triazin-2-yl)]amino}stilbene-2-2' disulophonate, disodium 4,4'-bis{[(4-anilino-6-morpholino-1,3,5-triazin-2-yl)]amino} stilbene-2-2' disulphonate, and disodium 4,4'-bis(2-sulphostyryl)biphenyl.

The total amount of the fluorescent agent or agents used in the composition is preferably of from 0.0001 to 0.5 weight-%, more preferably from 0.005 to 2 weight-%, most preferably from 0.05 to 0.25 weight-%.

Within a preferred embodiment, the inventive composition preferably comprises at least one further enzyme. If present, then the level of each enzyme in the laundry composition of the invention is from 0.0001 weight-% to 0.1 weight-%, preferably of from 0.005 to 0.1 weight-%. Further enzymes are preferably selected from the group of proteases, alpha-amylases, other cellulases (cellulases other than those specified in the invention as defined by the claims as filed), lipases, peroxidases/oxidases, pectate lyases, mannanases or mixtures thereof.

Within a particularly preferred embodiment, the at least one further enzyme is selected from proteases, alpha-amylases and lipases.

Within a preferred embodiment, the inventive composition preferably comprises at least one builder preferably selected from calcium sequestrant materials, precipitating materials, calcium ion-exchange materials and mixtures thereof.

Preferred calcium sequestrant builder materials include alkali metal polyphosphates, such as sodium tripolyphosphate and organic sequestrants, such as ethylene diamine tetra-acetic acid.

Preferred precipitating builder materials include sodium orthophosphate and sodium carbonate.

Preferred calcium ion-exchange builder materials include the various types of water-insoluble crystalline or amorphous aluminosilicates, of which zeolites are well known representatives, e.g. zeolite A, zeolite B (also known as zeolite P), zeolite C, zeolite X, zeolite Y and also the zeolite P-type as described in EP-A-0,384,070.

Within a preferred embodiment, the inventive composition preferably comprises from 0.001 to 65 weight-%, preferably of from 0.01 to 50 weight-%, further preferred of from 0.1 to 35 weight-% and most preferred of from 0.5 to 30 weight-% of a builder or complexing agent such as ethylenediaminetetraacetic acid, diethylenetriamine-pentaacetic acid, alkyl- or alkenylsuccinic acid or nitrilotriacetic acid.

Preferably the laundry cleaning formulation is a non-phosphate built laundry detergent formulation, i.e., contains less than 1 weight-%, preferably less than 0.8 weight-%, further preferred less than 0.5 weight-% of phosphate wherein ranges of from 0.001 to 5 weight-% and from 0.001 to 1 weight-% are also preferred.

Within a preferred embodiment, the inventive composition preferably comprises one or more polymers. Example polymers are polyethyleneimine, poly(vinylpyrrolidone), poly(vinylpyridine-N-oxide), poly(vinylimidazole), carboxymethylcellulose, poly(ethylene glycol), poly(vinyl alcohol), polycarboxylates such as polyacrylates, maleic/acrylic acid copolymers and lauryl methacrylate/acrylic acid copolymers. A preferred polymer is polyethyleneimine.

The present invention also pertains to a method of laundering white fabric, the method comprising contacting the fabric with an aqueous solution comprising a composition as defined within the present application. Within a preferred embodiment the contacting of the fabric with the aqueous solution occurs at 60 °C or less, 50 °C or less, 40 °C or less, 30 °C or less, preferably at 25 °C or less and most preferred at 22 °C or less.

As described herein, the cellulases in the compositions of the invention may be stable at higher temperatures, but show good activity at lower temperatures. Lower temperature washes may be preferable as they are more environmentally friendly, less expensive and, typically, less likely to damage fabrics and trims.

### Examples

The present invention is now described by the following examples and figures. The examples and figures are for illustrative purposes only and are not to be understood as limiting the invention.

List of Figures
- Fig. 1: shows the results of example 4: a comparison of the dL*-value of SEQ ID NO: 1 and SEQ ID NO: 11
- Fig. 2: shows the results of example 5: applying the different cellulase variants resulted in different L+-values, indicating different ARD-effect of the inventive cellulases SEQ ID NO: 15, SEQ ID NO: 13 and SEQ ID NO 17.
- Fig. 3: shows the results of example 6: ARD wash activity of SEQ ID NO: 11 compared to Celluclean® for carpet soil
- Fig. 4: shows the results of example 8: Applying SEQ ID NO: 11 with a concentration of 0.625 mg/L at 20°C resulted in a dL* value of 4.5

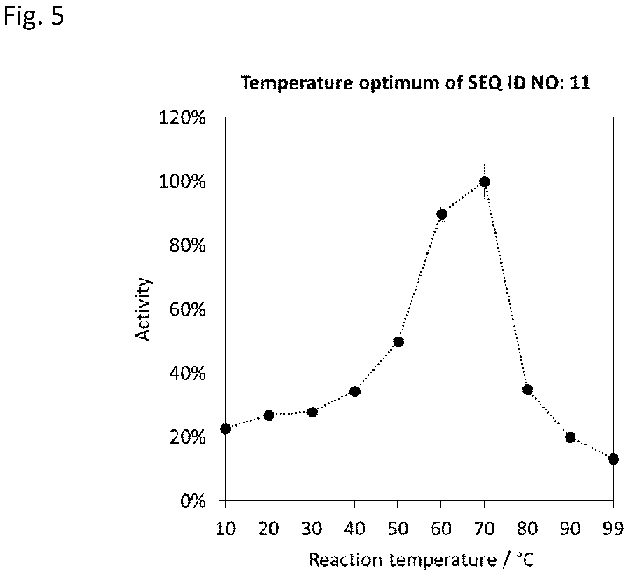

- Fig. 5: shows the results of example 9: The temperature optimum of SEQ ID NO: 11

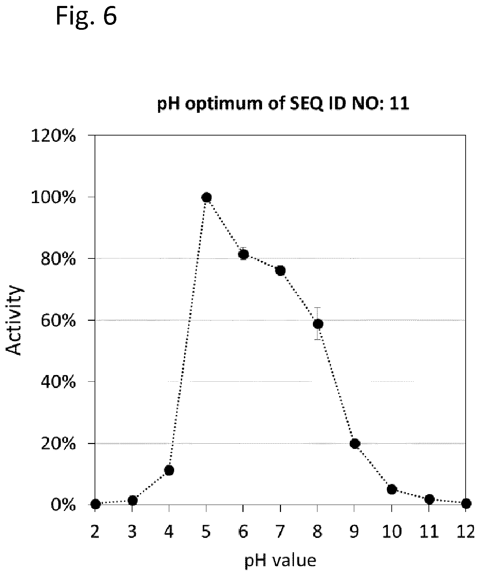

- Fig. 6: shows the results of example 10: The pH optimum of SEQ ID NO: 11

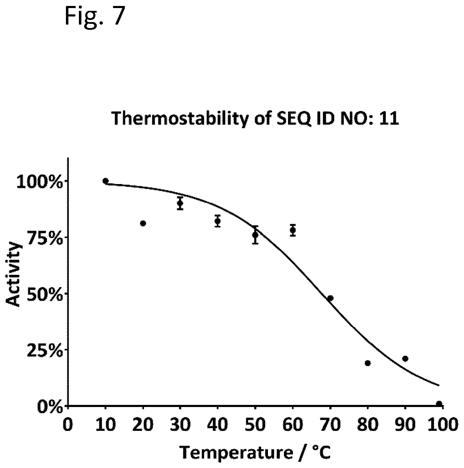

- Fig. 7: shows the results of example 11: The thermostability of SEQ ID NO: 11
- Fig. 8: shows the results of example 12: a comparison of the dL*-value of SEQ ID NO: 11, Celluclean® and/or AV50
- Fig. 9: shows the results of example 13: persistency of whiteness effect in multiple machine wash studies

### Example 1: Cellulase expression of Cellulases in Pichia pastoris

For the expression of the proteins (the following methods refers to SEQ ID NO: 15 and SEQ ID NO: 1 as examples), the corresponding genes (SEQ ID NO: 2 and SEQ ID NO: 16) were cloned into standard vectors at first. Therefore, the two genes were amplified via PCR with primers having a restriction site as overhang. PCR products and vectors (pGAPZa A, pPICZa A; Invitrogen™), digested with KpnI and XbaI, were ligated according to suppliers manual. The resulted vectors are listed in table 1.

**Table 1: Plasmid names and description**

| **plasmid name** | **description** |
|---|---|
| pGAP-SEQ ID NO: 1 | pGAPZα A with SEQ ID NO: 1 |
| pAOX-SEQ ID NO: 1 | pPICZα A with SEQ ID NO: 1 |
| pGAP-SEQ ID NO: 15 | pGAPZα A with SEQ ID NO: 15 |
| pAOX-SEQ ID NO: 15 | pPICZα A with SEQ ID NO: 15 |

After linearization with BglI, the vectors were transformed into X-33 strain (Invitrogen™) according to suppliers manual. For each plasmid, 96 transformants were screened for high protein production in deep-well plates. From each transformant series the best 5-10 clones were cultivated in 300 ml shake flasks (50 ml YP base medium with 1% glycerol; 27°C, 250 rpm, 80 % humidity; 2 ml shots of glycerin or methanol after 40 h, 48h, 66 h and 72 h; harvest after 96 h) for verification. The activity of the proteins was determined with Azo-CMC Assay (Megazyme, Ireland) at the end.

### Example 2: Determination of enzyme concentration by gel quantification of target bands

SEQ ID NO: 1, SEQ ID NO: 11, SEQ ID NO: 13, SEQ ID NO: 15 and SEQ ID NO: 17 fermentation supernatants were quantified via an in house SDS gel quantification method using an external protein calibration curve. Enzyme samples were applied to an SDS gel which was subsequently stained with Sypro Ruby (Thermo Fisher: S12000). The gel image was recorded on a standard Bio-Rad gel documentation instrument. Image analysis was performed using ImageLab software (Bio-Rad). Protein concentration was determined by signal integration of the target protein's specific SDS gel band using the external protein calibration curve on the same SDS gel (e.g. BSA; bovine serum albumin).

### Example 3: Cellulase quantification in fermentation supernatants using specific enzyme activities

SEQ ID NO: 1 and SEQ ID NO: 11 were purified to homogeneity by Ni-NTA purification followed by size exclusion chromatography using a Superdex 75 10/300GL column. The protein sequences were determined experimentally by intact mass determination and N-terminal sequencing. Protein quantification of the homogenous samples was performed by HPLC with UV280 signal detection using the molar extinction coefficient calculated from the experimentally determined protein sequence. In brief, HPLC runs were performed as follows. The protein was applied to an end capped Nucleosil C4 column and eluted by a linear gradient of buffer A (90% water, 10% acetonitrile, 0.1% TFA) and buffer B (100% acetonitrile, 0.1% TFA). The peak UV280 signal was integrated to calculate the protein concentration. Volumetric enzyme activities of purified SEQ ID NO: 1 and SEQ ID NO: 11 samples, or crude protein supernatants were measured using a modified, 96 well enabled Cellazyme® C assay (Megazyme). Specific and molar activities of size exclusion chromatography purified SEQ ID NO: 1 and SEQ ID NO: 11 were used to calculate target protein concentrations in the crude fermentation supernatants.

### Example 4: ARD (Anti-re-deposition) wash activity of cellulases

The activity of cellulases was tested in an ARD washing test in Tergotometer scale. White cotton swatches (T460-40, center for testmaterials B.V. 3133 KT Vlaardingen, The Netherlands) were washed 4 times 20 minutes in 800 mL at 40°C. The wash liquor consisted of the detergent AATCC WOB 93 in a dosage of 1.5 g/L in water with a defined French hardness of 27, Ca:Mg 2:1. To each wash cycle 8 new cotton swatches soiled with carbon black and olive oil (8x10 cm; 101 swissatest Testmaterialien AG, 9015 St Gallen, Switzerland) were added as soil ballast. The liquor cloth ratio was set to 25 by adding cotton fabric 80 A and 10 A (wfk Testgewebe GmbH, 41379 Brüggen, Germany) as ballast. The L*a*b* values of the white cotton swatches were recorded after drying the textile at the air using a spectrophotometer with D65, 10°(ColorFlex EZ, Hunterlab). The instrument was calibrated prior to the measurement with a supplied white standard. The dL* value was calculated by subtraction of the L* of a blank control without enzyme from L* of cotton swatches treated with cellulase. The dL* reflects the whiteness of a fabric, a higher dL* therefore indicates a higher ARD-effect of enzyme. Enzymes were dosed as mg of active enzyme protein (AEP). AEP content of each preparation was calculated based on specific enzyme activities. The protein quantification was performed as described in example 3. Dosage of the enzyme preparations was 0.625 mg of active enzyme protein per liter of wash liquor and control sample contained no enzyme.

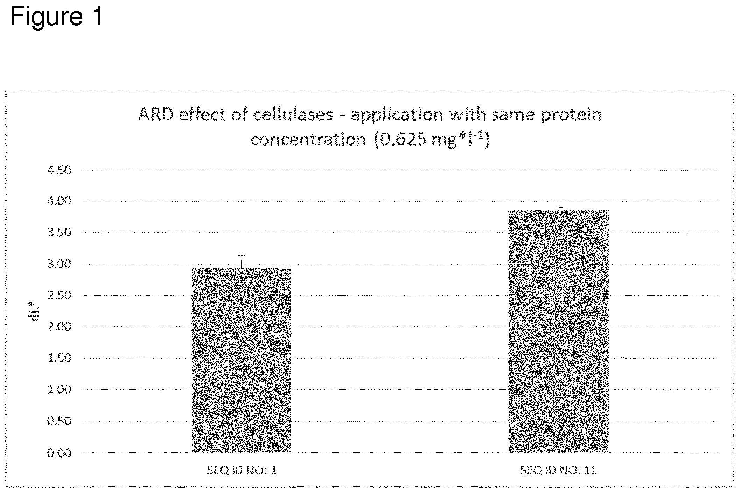

Results are shown in Figure 1. Applying the enzymes with the same protein concentration resulted in a higher dL* value for SEQ ID NO: 11 (3.85) compared to SEQ ID NO: 1 (2.94) indicating an increased ARD-effect for SEQ ID NO: 11.

### Example 5: ARD (Anti Re-Deposition) wash activity of cellulase variants

The activity of cellulases was tested in an ARD washing test in Tergotometer scale. White cotton swatches (T460-40, center for testmaterials B.V. 3133 KT Vlaardingen, The Netherlands) were washed 4 times 20 minutes in 800 mL at 40°C. The wash liquor consisted of detergent AATCC WOB 93 in a dosage of 1.5 g/L in water with set French hardness 27, Ca:Mg 2:1. To each wash cycle 8 new cotton swatches soiled with carbon black and olive oil (8x10 cm; 101 swissatest Testmaterialien AG, 9015 St Gallen, Switzerland) were added as soil ballast. The liquor cloth ratio was set to 25 by adding cotton fabric 80 A and 10 A (wfk Testgewebe GmbH, 41379 Brüggen, Germany) as ballast. The L*a*b* values of the white cotton swatches were recorded after drying the textile at the air using a spectrophotometer with D65, 10°(ColorFlex EZ, Hunterlab). The instrument was calibrated prior to the measurement with a supplied white standard. The dL* value was calculated by subtraction of the L* of a blank control without enzyme from L* of cotton swatches treated with cellulase. The dL* reflects the whiteness of a fabric, a higher dL* therefore indicates a higher ARD-effect of enzyme. Enzymes were dosed as mg of active enzyme protein (AEP). AEP content of each preparation was calculated based on a SDS-PAGE, which was applied for protein quantification (example 2). Dosage of the enzyme preparations was 1.5 mg of active enzyme protein per liter of wash liquor and control sample contained no enzyme.

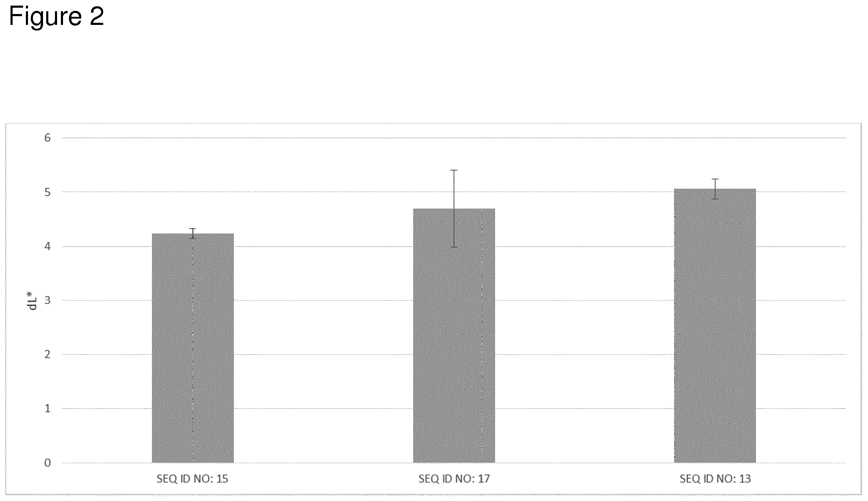

Results are shown in Figure 2. Applying the different cellulase variants resulted in different L+-values, indicating different ARD-effect of the cellulase variants.

### Example 6: ARD (Anti Re-Deposition) wash activity of enzymes with carpet soil

The wash activity of cellulases was tested in an ARD washing test in Tergotometer scale. White cotton swatches (T460-40, center for testmaterials B.V. 3133 KT Vlaardingen, The Netherlands) were washed 7 times 20 minutes in 800 mL at 35°C. The wash liquor consisted of the detergent AATCCliqD in a dosage of 2 g/L in water with French hardness set to 26, Ca:Mg 2:1. Liquor cloth ratio was set to 29 by adding cotton fabric 80 A and 10 A (wfk Testgewebe GmbH, 41379 Brüggen, Germany) as ballast. As soil, 10 g/L carpet soil wfk 09W (wfk Testgewebe GmbH, 41379, Brüggen, Germany) was added into the wash liquor at each wash cycle.

Enzymes were dosed in a concentration of 0.625 mg/L (AEP; active protein; example 2). The L*a*b* values of the white cotton swatches were recorded after drying the textile at the air using a spectrophotometer with D65, 10°(ColorFlex EZ, Hunterlab). The instrument was calibrated daily prior to each measurement with the supplied white standard (Hunterlab). The dL* value was calculated by subtraction of the L* of a blank control without enzyme from L* of cotton swatches treated with cellulase. The dL* gives the whiteness of a fabric, a higher dL* therefore indicates a higher ARD-effect of enzyme.

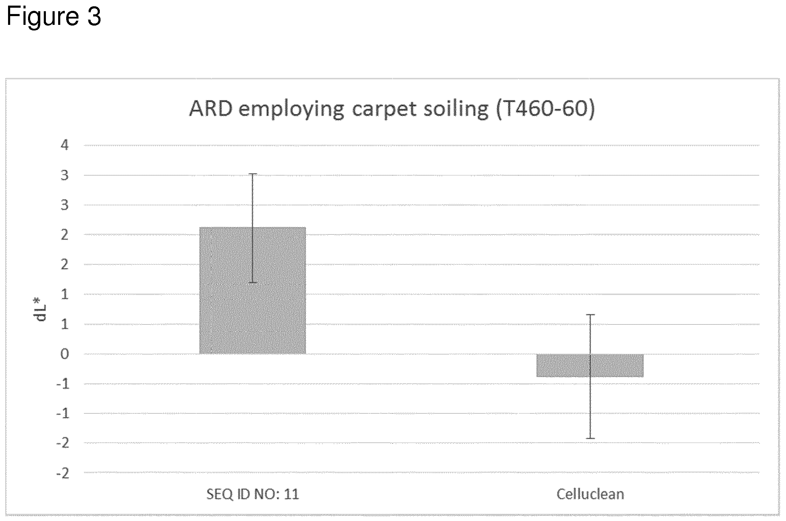

Results are shown in Figure 3. SEQ ID NO: 11 shows a positive ARD effect with dL* of ∼2 compared to fabric washed without enzyme treatment. Celluclean® 5000L on the other hand and shows on average a minimal negative ARD wash effect with carpet soil.

### Example 7: Launder-O-meter tests of a cellulase with liquid detergent application

The tests were conducted as disclosed in WO 2016 066896. The cellulase (SEQ ID NO: 11) was produced in *Pichia pastoris,* as described in example 1, and tested for their performance with AATCCliq. detergent at 40°C. The monitor E-253 was used for the demonstration of the de-pilling effect representing used cotton textiles. The test fabrics were cut into swatches (approx. 29 cm x 15 - 16.5 cm, total weight of two swatches approx. 24 g) containing full width stripes of each color (black, red, green, blue).

Cellulase treatments were performed in an Atlas LP-2 Launder-O-meter (SDS Atlas, Rock Hill, SC 29732, USA) as follows. The Launder-O-meter was first pre-heated to 40°C. Subsequently, 60 g of steel balls (diameter 0.6 cm) and 240 ml of wash liquor and diluted enzyme (<1 .0 ml) were added into 1.2 liter containers. After that, one swatch of E-253 was placed in containers and the Launder-O-meter was run at 40°C for 60 min.

Enzymes were dosed as mg of active enzyme protein (AEP). AEP content of each preparation was calculated based on a SDS-PAGE which was applied for protein quantification (example 2). Dosage of the enzyme preparations was 0.4 mg of active enzyme protein per liter of wash liquor and control sample contained no enzyme. The wash liquor contained 5 g of AATCCliq. per litre of synthetic tap water (16°dH). The preparation of the synthetic tap water with a hardness of 16°dH was prepared as described in WO 2016 066896 in example 4. After the cellulase treatment in the Launder-O-meter, the swatches were first rinsed separately under running water (ambient temperature ∼20°C) and then dried in a spin-dryer (THOMAS, Neunkirchen; Type: 776 SEL 202) for 5 minutes.

The cellulase performance in detergent application was evaluated by measuring the color of as reflectance values using a spectrophotometer (ColorFlex EZ, Hunterlab) using L*a*b* color space coordinates. The color of each 4 stripes of test monitors was measured after 5 washing cycles. Decrease of lightness (L*), i.e. increase of darkness compared to treatment without cellulase, was used as an indication of cellulase effect. When the surface fibers and fibrils protruding from the yarn forming pills and giving the fabric a greyish look are removed by cellulase, the lightness of the fabric decreases, and the surface of the fabric appears darker and colors get brighter (WO 2016 066896).

Cellulase performance was calculated according to WO 2016 066896 in example 4. The sum of the L*-values of all 4 test strips on the monitor appeared to be negative (-2.50). As described above, a negative L*-value indicates the removal of fibers and fibrils protruding from the yarn forming pills, giving the fabric a greyish look. The spectrophotometrical results were also confirmed by visual evaluation.

### Example 8: ARD (Anti-re-deposition) wash activity of a cellulase at 20°C

The activity of of SEQ ID NO: 11 was tested in an ARD washing test in Tergotometer scale at 20°C. White cotton swatches WK05, wfk Testgewebe GmbH, 41379 Brüggen, Germany) were washed 20 minutes in 800 mL at 20°C. The wash liquor consisted of the detergent IKEA12 in a dosage of 0.72 g/L in water with a defined French hardness of 26, Ca:Mg 2:1. To each wash cycle 0.04 g/L were added.

**Composition of IKEA 12**

| **Ingredient** | **Wt.%** |
|---|---|
| Demin water | To 100 |
| TEA | 1.95 |
| NaOH | 1.28 |
| Gulf Farabi LAS (linear alkylbenzene sulfonate) | 11.33 |
| Texapon N701 (1EO) (nonionic surfactant) | 7.17 |
| Proxel GXL (1, 2-benzisothiazolin-3-one) | 0.02 |
| Neolone 950 (Methylisothiazolone) | 0.01 |
| NaCl | 1.00 |

The liquor cloth ratio was set to 33 by adding a woven cotton fabric as ballast. The L*a*b* values of the white cotton swatches were recorded after drying the textile at 25°C; 10% humidity overnight in a spectrophotometer with D65, 10°(ColorFlex EZ, Hunterlab). The instrument was calibrated prior to the measurement with a supplied white standard. The dL* value was calculated by subtraction of the L* of a blank control without enzyme from L* of cotton swatches treated with cellulase. The dL* reflects the whiteness of a fabric, a higher dL* therefore indicates a higher ARD-effect of enzyme. Enzymes were dosed as mg of active enzyme protein (AEP). AEP content of each preparation was calculated based on the method described in example 2. Dosage of the enzyme preparations was 0.625 mg of active enzyme protein per liter of wash liquor and control sample contained no enzyme.

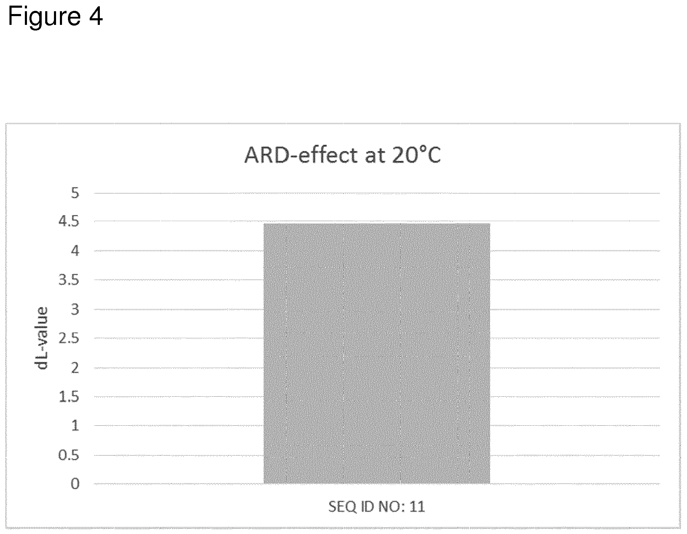

Results are shown in Figure 4. Applying SEQ ID NO: 11 with a concentration of 0.625 mg/L resulted in a dL* value of 4.5.

### Example 9: Determination of temperature optimum of SEQ ID NO: 11

For the determination of the temperature optimum 100 µL enzyme solutions in 100 mM sodium acetate buffer pH 5 were mixed with 100 µL 1% aqueous carboxymethyl-cellulose solution and incubated for 10 minutes at 10°C, 20°C, 30°C, 40°C, 50°C, 60°C, 70°C, 80°C, 90°C and 99°C, shaking. The reactions were stopped by incubation of the reaction mixes at 95°C for 5 minutes. Activity was determined by measurement of the liberated reducing ends using the p-hydroxybenz-hydrazide assay.

For reducing end determination, a 5% (w/v) p-hydroxybenz-hydrazide stock solution in 0.5 M HCl was diluted 1:3 in 0.5 M NaOH to yield the p-hydroxybenz-hydrazide working solution. 50 µL of sample was mixed with 150 µL of working solution and the reaction was incubated for 5 min at 95°C. After cooling to 4°C the absorbance at 410 nm was determined and liberated reducing ends were calculated using a glucose calibration curve.

The results show, that SEQ ID NO: 11 guarantees activity at all temperatures relevant for cleaning and washing applications. Even at low temperatures an activity of more than 20% is maintained.

### Example 10: Determination of pH optimum of SEQ ID NO: 11

For the determination of pH optimum, an enzyme stock solution was diluted in 120 mM Britton-Robinson universal buffers adjusted to pH 2, 3, 4, 5, 6, 7, 8, 9, 10, 11 and 12, respectively. 100 µL of these dilutions were mixed with 100 µL aqueous 1% carboxymethyl-cellulose solution and incubated for 10 minutes at 70°C, shaking. The reactions were stopped by incubation of the reaction mixes at 95°C for 5 minutes. Activity was determined by measurement of the liberated reducing ends using the p-hydroxybenz-hydrazide assay as described in Example 9.

The results show that SEQ ID NO: 11 guarantees activity over a broad pH range and still shows very good activity at high/basic pH values (up to pH 9) which are relevant for washing and cleaning applications.

### Example 11: Determination of temperature and pH optimum, and thermostability of SEQ ID NO: 11

For the determination of temperature stability 100 µL enzyme solutions in 100 mM sodium acetate buffer pH 5 were incubated for 30 min at 10°C, 20°C, 30°C, 40°C, 50°C, 60°C, 70°C, 80°C, 90°C and 99°C. Solutions were cooled to 4°C. Subsequently, 100 µL of aqueous 1% carboxymethyl-cellulose solution were added and the samples were incubated at 50°C for 10 minutes. The reactions were stopped by addition of 40 µL 1 M sodium carbonate solution. Activity was determined by measurement of the liberated reducing ends using the p-hydroxybenz-hydrazide assay as described in Example 9.

The results show that SEQ ID NO: 11 guarantees excellent temperature stability showing activity for a broad temperature range.

### Example 12: comparison of SEQ ID NO:11, Celluclean® and/or AV50 regarding dL* and proof of synergistic effect

### Tergotometer wash studies

The conditions used in the wash process were as follows:
Temperature: 20 °C
Enzyme concentration: 0.625ppm
Product dose: 0.72gpl IKEA12
Soil dose: 0.04gpl carbon black
Shading dye dose: 0.006% AV50
Wash time: 1 x 20 minute wash
Rinse time: 1 x 10 seconds rinse

This example shows that the combination of cellulase SEQ ID NO: 11 with a shading dye (AV50) provides a synergistic effect in terms of whiteness over and above the effects provided separately by SEQ ID NO:11, Celluclean® and shading dye.

| | **Whiteness value (normalized versus base formulation)** |
|---|---|
| + Shading Dye (AV50) | 1.9 ± 0.3 |
| + Celluclean® | 2.6 ± 0.3 |
| + SEQ ID NO: 11 | 3.4 ± 0.3 |
| + AV50 + Celluclean (Calculated) | 4.5 ± 0.2 |
| + AV50 + Celluclean (Measured) | 4.0 ± 0.4 |
| + AV50 + SEQ ID NO: 11 (Calculated) | 5.3 ± 0.2 |
| + AV50 + SEQ ID NO: 11 (Measured) | 7.2 ± 0.3 |

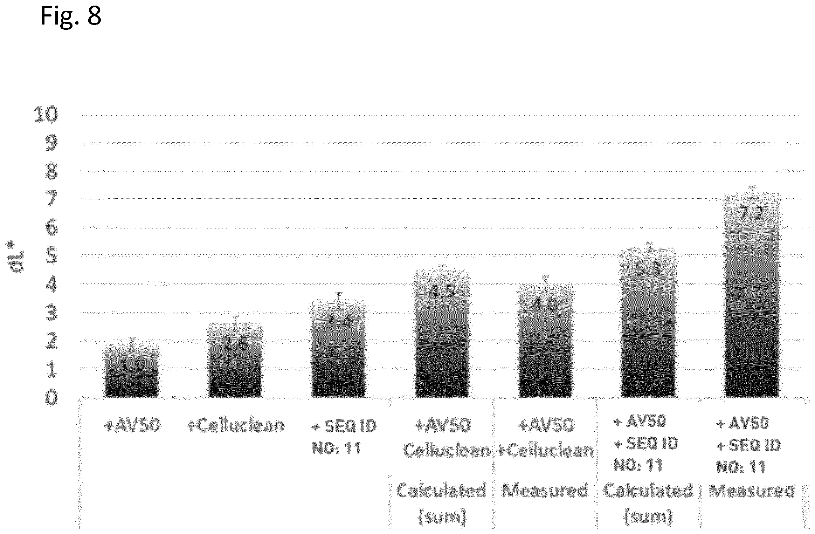

It is notable that the combination of cellulase SEQ ID NO: 11 with a shading dye (AV50) provides a synergistic benefit (measured versus calculated), in comparison to the Celluclean® combination with shading dye, where the measured value is lower than the calculated whiteness value. The results are shown in figure 8.

### Example 13: persistency of whiteness effect

### Multiple machine wash studies

Cotton Monitors used were pre aged by washing them 20 times with OMO powder. 3 replicates per wash point of 1, 3, 5, 10, 15, 20, 25 and 30 washes were used. Soil Monitors used comprised of 3 x E101 monitors. Fabrics were washed in a range of formulations +/- Celluclean® and AV50 (shading dye) to assess if there is any improvement to redeposition due to the enzyme being present. Machines used for the study were Asian TLA 45L. Washes were carried out in 40°C 26 FH (2:1 Ca:Mg) 45L wash liquor and the ballast load used was 1.5kg.

| | **OMO** | **OMO + AV50** | **OMO + Celluclean®** | **OMO + Cellulase SEQ. ID 11** | **OMO + Cellucle an® + AV50** | **OMO + Cellulase SEQ. ID 11 + AV50** |
|---|---|---|---|---|---|---|
| **1 Wash** | - 8.4 | - 7.9 | - 4.8 | - 5.2 | - 6.4 | - 7.2 |
| **3W** | - 13.6 | - 13.5 | - 7.3 | - 6.9 | - 8.8 | - 8.0 |
| **5W** | - 15.1 | - 16.3 | - 9.9 | - 8.9 | - 10.9 | - 9.6 |
| **10W** | - 21.4 | - 21.7 | - 19.2 | - 15.1 | - 19.8 | - 15.3 |
| **15W** | - 23.6 | - 23.7 | - 19.0 | - 15.9 | - 20.6 | - 16.3 |
| **20W** | - 25.1 | - 24.1 | - 19.2 | - 17.2 | - 19.9 | - 16.8 |
| **25W** | - 25.4 | - 25.1 | - 20.0 | - 17.4 | - 19.9 | - 17.8 |
| **30W** | - 26.6 | - 26.8 | - 21.3 | - 19.1 | - 20.5 | - 18.2 |

| **OMO Ingredients** | **OMO (%)** |
|---|---|
| Sodium LAS | 8.00 |
| Sodium Silicate | 7.11 |
| Soda Ash | 22.89 |
| Sodium Sulphate | 59.39 |
| CP5/Polymer | 0.36 |
| S.C.M.C. | 0.13 |
| Tinopal CBSx/DSBP | 0.02 |
| Moisture | 1.78 |
| Salts & NDOM | 0.28 |
| Total | 100.00 |
| AD | 8.00 |
| Alkalinity | 30.00 |

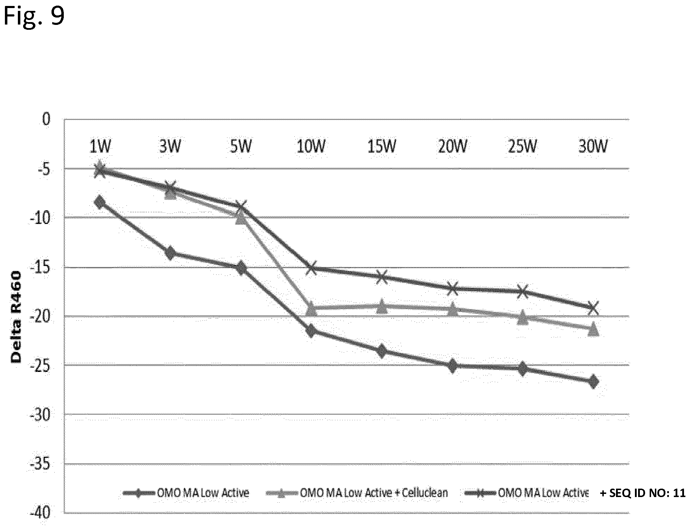

This example shows that the enhanced whiteness effect persisted over many (30) washes for cellulase SEQ ID NO: 11 in comparison to Celluclean®. The effect was shown on knitted aged cotton and the base formulation was OMO powder. The values shown at Δ460 Reflectance values normalised from unwashed fabric, so they are given as negative values. A smaller negative value means the washed fabric is closer to pristine unwashed value. Figure 9 shows some selected results (for OMO, OMO + Celluclean®, and OMO + SEQ ID NO: 11) from the table below in graphical format.

Sequences identified within the present invention:
SEQ ID NO: 1 sequence comprising a catalytic domain motif [STA]-T-R-Y-[FYW]-D-x(5)-[CA]
SEQ ID NO: 2 corresponding nucleotide sequence to SEQ ID NO: 1
SEQ ID NO: 3 carbohydrate binding domain
SEQ ID NO: 4 corresponding nucleotide sequence to SEQ ID NO: 3
SEQ ID NO: 5 linker sequence
SEQ ID NO: 6 corresponding nucleotide sequence to SEQ ID NO: 5
SEQ ID NO: 7 linker sequence
SEQ ID NO: 8 corresponding nucleotide sequence to SEQ ID NO: 7
SEQ ID NO: 9 preferred catalytic domain motif T-T-R-Y-W-D-C-C-K-P-S-C
SEQ ID NO: 10 corresponding nucleotide sequence to SEQ ID NO: 9
SEQ ID NO: 11 preferred cellulase according to the present invention
SEQ ID NO: 12 corresponding nucleotide sequence to SEQ ID NO: 11
SEQ ID NO: 13 preferred cellulase according to the present invention
SEQ ID NO: 14 corresponding nucleotide sequence to SEQ ID NO: 13
SEQ ID NO: 15 preferred cellulase according to the present invention
SEQ ID NO: 16 corresponding nucleotide sequence to SEQ ID NO: 15
SEQ ID NO: 17 preferred cellulase according to the present invention
SEQ ID NO: 18 corresponding nucleotide sequence to SEQ ID NO: 17
SEQ ID NO: 19 preferred carbohydrate binding domain tag
SEQ ID NO: 20 corresponding nucleotide sequence to SEQ ID NO: 19

## Claims

1. Cellulase comprising the catalytic domain motif [STA]-T-R-Y-[FYW]-D-x(5)-[CA] and a carbohydrate binding domain with a sequence identity of at least 80% to SEQ ID NO: 3 or a carbohydrate binding domain comprising the tag motif V-[PSC]-[DQEN]-S-G-G-P-G-P-G-P-G-P-G-P and wherein the cellulase has a sequence identity of at least 99% to SEQ ID NO: 11, SEQ ID NO: 13, SEQ ID NO: 15 or SEQ ID NO: 17.

2. Cellulase according to claim 1, wherein the catalytic domain motif is T-T-R-Y-[FYW]-D-x(5)-[CA].

3. Cellulase according to any of claims 1 or 2, wherein the catalytic domain motif is T-T-R-Y-W-D-x(5)-C.

4. Cellulase according to any of claims 1 to 3, wherein the catalytic domain motif is T-T-R-Y-W-D-C-C-K-P-S-C.

5. Cellulase according to any of the preceding claims, comprising a sequence with at least 80% sequence identity to SEQ ID NO: 1.

6. Cellulase according to any of the preceding claims, further comprising a linker.

7. Cellulase according to claim 6, wherein the linker has a sequence identity of at least 80% to SEQ NO: 5.

8. Cellulase according to claim 6, wherein the linker has a sequence identity of at least 80% to SEQ NO: 7.

9. Cellulase according to any of claims 6 or 7, wherein the linker comprises an amino acid sequence of [AGSVT](5,65).

10. Cellulase according to claim 8, wherein the linker comprises an amino acid sequence of ([SG]-P)(5,10).

11. Use of the cellulase as defined in any of claims 1 to 10 in textile, feed, food, biomass hydrolysis, plant oil refining, detergent and pulp and paper processing applications.

12. Use of the cellulase according to claim 11 as a biofinishing agent such as a depilling agent or a defuzzing agent; a biostoning agent; a fabric care agent such as a color clarification agent, an anti greying agent, a softness agent, an antipilling agent; a laundry agent such as an an anti-redeposition agent, a soil removal agent; a malting and brewing agent such as a filtration agent, a color extraction agent; a bakery agent such as a texture agent; a feed agent such as a viscosity reduction agent; a fruit processing agent such as a release antioxidants agent, a pressing agent, a color extraction agent, a clarification of juice agent; a plant oil extraction agent; a pulp and paper processing agent such as a deinking agent, a fiber brightness agent, a drainage agent and/or a bleaching agent.

13. Composition comprising at least one cellulase as defined in any of claims 1 to 10.

14. Composition according to claim 13 further comprising at least one surfactant, builder, co-builder, anti-redeposition agent, dispersant, optical brightener, bleaching agent, dye, enzyme, chelator, polymer, pH buffering agent, filler, flocculant, fabric softener, foam booster, perfume, suds supressor, bacteriocide, fungicide, enzyme inhibitor, stabilizer, solubilizer, antioxidant, anti-corrosion agent and/or pigment.

## Patentansprüche

1. Cellulase, die das katalytische Domänen-Motiv [STA]-T-R-Y-[FYW]-D-x(5)-[CA] und eine Kohlenhydrat-bindende Domäne mit einer Sequenzidentität von mindestens 80 % zu SEQ ID Nr.: 3 oder eine das Tag-Motiv V-[PSC]-[DQEN]-S-G-G-P-G-P-G-P-G-P-G-P umfassenden Kohlenhydrat-bindende Domäne umfasst und wobei die Cellulase eine Sequenzidentität von mindestens 99 % zu SEQ ID Nr.: 11, SEQ ID Nr.: 13, SEQ ID Nr.: 15 oder SEQ ID Nr.: 17 aufweist.

2. Cellulase nach Anspruch 1, wobei das katalytische Domänen-Motiv T-T-R-Y-[FYW]-D-x(5)-[CA] ist.

3. Cellulase nach einem der Ansprüche 1 oder 2, wobei das katalytische Domänen-Motiv T-T-R-Y-W-D-x(5)-C ist.

4. Cellulase nach einem der Ansprüche 1 bis 3, wobei das katalytische Domänen-Motiv T-T-R-Y-W-D-C-C-K-P-S-C ist.

5. Cellulase nach einem der vorhergehenden Ansprüche, die eine Sequenz mit einer Sequenzidentität von mindestens 80 % zu SEQ ID Nr.: 1 aufweist.

6. Cellulase nach einem der vorhergehenden Ansprüche, die weiterhin einen Linker umfasst.

7. Cellulase nach Anspruch 6, wobei der Linker eine Sequenzidentität von mindestens 80 % zu SEQ ID Nr.: 5 aufweist.

8. Cellulase nach Anspruch 6, wobei der Linker eine Sequenzidentität von mindestens 80 % zu SEQ ID Nr.: 7 aufweist.

9. Cellulase nach einem der Ansprüche 6 oder 7, wobei der Linker eine Aminosäuresequenz [AGSVT](5, 65) umfasst.

10. Cellulase nach Anspruch 8, wobei der Linker eine Aminosäuresequenz ([SG]-P)(5,10) umfasst.

11. Verwendung der in einem der Ansprüche 1 bis 10 definierten Cellulase in Textil-, Futter-, Nahrungsmittel-, Biomassehydrolyse-, Pflanzenölraffinations-, Detergenzien- und Zellstoff- und Papierverarbeitungsanwendungen.

12. Verwendung der Cellulase nach Anspruch 11 als Bio-Veredelungsmittel wie ein Enthaarungsmittel oder ein Entfusselungsmittel; ein Bio-Stoning-Mittel; ein Textilpflegemittel wie ein farberneuerndes Mittel, ein Vergrauungsschutzmittel, Weichspülmittel, Antipilling-Mittel; ein Wäschewaschmittel wie ein Vergrauungsinhibitor, ein schmutzentfernendes Mittel; ein Mälz- und Braumittel wie ein Filtriermittel, ein Farbextraktionsmittel; ein Backmittel wie ein Texturmittel; ein Futtermittel wie ein viskositätsverminderndes Mittel; ein Obstverarbeitungsmittel wie ein Antioxidantientrennmittel, ein Presshilfsmittel, ein Farbextraktionsmittel, ein Saftklärungsmittel; ein Pflanzenölextraktionsmittel; ein Zellstoff- und Papierverarbeitungsmittel wie ein Entfärbungsmittel, ein Faseraufhellungsmittel, ein Entwässerungsmittel und/oder ein Bleichmittel.

13. Zusammensetzung, die mindestens eine in einem der Ansprüche 1 bis 10 definierte Cellulase umfasst.

14. Zusammensetzung nach Anspruch 13, die weiterhin mindestens ein Tensid, einen Builder, Cobuilder, Vergrauungsinhibitor, ein Dispergiermittel, einen optischen Aufheller, ein Bleichmittel, einen Farbstoff, ein Enzym, einen Chelatbildner, ein Polymer, ein Mittel zur Pufferung des pH-Werts, Füllmittel, Flockungsmittel, einen Weichspüler, Schaumverstärker, ein Parfüm, einen Schaumunterdrücker, ein Bakterizid, Fungizid, einen Enzyminhibitor, Stabilisator, Löslichmacher, ein Oxidationsschutzmittel, Korrosionsschutzmittel und/oder ein Pigment umfasst.

## Revendications

1. Cellulase comprenant le motif du domaine catalytique [STA]-T-R-Y-[FYW]-D-x(5)-[CA] et un domaine de liaison à un glucide avec une identité de séquence d'au moins 80% avec la SEQ ID n° : 3 ou un domaine de liaison à un glucide comprenant le motif d'étiquette V-[PSC]-[DQEN]-S-G-G-P-G-P-G-P-G-P-G-P et la cellulase ayant une identité de séquence d'au moins 99% avec la SEQ ID n° : 11, la SEQ ID n° : 13, la SEQ ID n° : 15 ou la SEQ ID n° : 17.

2. Cellulase selon la revendication 1, dans laquelle le motif du domaine catalytique est T-T-R-Y-[FYW]-D-x(5)-[CA].

3. Cellulase selon l'une quelconque des revendications 1 ou 2, dans laquelle le motif du domaine catalytique est T-T-R-Y-W-D-x(5)-C.

4. Cellulase selon l'une quelconque des revendications 1 à 3, dans laquelle le motif du domaine catalytique est T-T-R-Y-W-D-C-C-K-P-S-C.

5. Cellulase, selon l'une quelconque des revendications précédentes, comprenant une séquence avec une identité de séquence d'au moins 80% avec la SEQ ID n° : 1.

6. Cellulase, selon l'une quelconque des revendications précédentes, comprenant en outre un lieur.

7. Cellulase selon la revendication 6, dans laquelle le lieur a une identité de séquence d'au moins 80% avec la SEQ ID n° : 5.

8. Cellulase selon la revendication 6, dans laquelle le lieur a une identité de séquence d'au moins 80% avec la SEQ ID n° : 7.

9. Cellulase selon l'une quelconque des revendications 6 ou 7, dans laquelle le lieur comprend une séquence d'acides aminés de [AGSVT](5,65).

10. Cellulase selon la revendication 8, dans laquelle le lieur comprend une séquence d'acides aminés de ([SG]-P)(5,10).

11. Utilisation de la cellulase, telle que définie dans l'une quelconque des revendications 1 à 10, dans des applications textiles, d'aliments pour animaux, alimentaires, d'hydrolyse de biomasse, de raffinage d'huiles végétales, de détergents et de traitement de la pâte et du papier.

12. Utilisation de la cellulase, selon la revendication 11, comme agent de bio-finition tel qu'un agent de déboulochage ou un agent défrisant ; un agent de délavage biologique ; un agent d'entretien des tissus tel qu'un agent de clarification de la couleur, un agent anti-grisonnement, un agent adoucissant, un agent anti-boulochage ; un agent de blanchisserie tel qu'un agent anti-redéposition, un agent d'élimination de la saleté ; un agent de maltage et de brassage tel qu'un un agent de filtration, un agent d'extraction de la couleur ; un agent de boulangerie tel qu'un agent texturant ; un agent d'aliments pour animaux tel qu'un agent de réduction de la viscosité ; un agent de traitement de fruits tel qu'un agent de libération d'antioxydants, un agent de pressage, un agent d'extraction de la couleur, un agent de clarification de jus ; un agent d'extraction d'huiles végétales ; un agent de traitement de la pâte et du papier tel qu'un agent de désencrage, un agent de luminosité des fibres, un agent de drainage et/ou un agent blanchissant.

13. Composition comprenant au moins une cellulase telle que définie dans l'une quelconque des revendications 1 à 10.

14. Composition, selon la revendication 13, comprenant en outre au moins un tensioactif, un adjuvant, un co-adjuvant, un agent anti-redéposition, un agent dispersant, un azurant optique, un agent blanchissant, un colorant, une enzyme, un chélateur, un polymère, un agent de tamponnage du pH, un agent de charge, un floculant, un adoucissant textile, un agent moussant, un parfum, un suppresseur de mousse, un bactéricide, un fongicide, un inhibiteur d'enzyme, un stabilisateur, un agent solubilisant, un antioxydant, un agent anticorrosion et/ou un pigment.
